**Europäisches Patentamt**

**European Patent Office**

**Office européen des brevets**

(19)

(11) Publication number: **0 026 093**
**A1**

(12) # EUROPEAN PATENT APPLICATION

(21) Application number: **80303306.7**

(22) Date of filing: **19.09.80**

(51) Int. Cl.³: **G 01 N 21/85**

(30) Priority: **22.09.79 GB 7932947**

(43) Date of publication of application:
**01.04.81 Bulletin 81/13**

(84) Designated Contracting States:
**BE DE FR GB IT NL SE**

(71) Applicant: **The British Petroleum Company Limited**
**Britannic House Moor Lane**
**London EC2Y 9BU(GB)**

(72) Inventor: **Moore, Kevin Daniel**
**The British Petroleum Co. Ltd. Chertsey Road**
**Sunbury-on-Thames Middlesex TW16 7LN(GB)**

(72) Inventor: **Spearing, Michael Henry**
**The British Petroleum Co. Ltd. Chertsey Road**
**Sunbury-on-Thames Middlesex TW16 7LN(GB)**

(74) Representative: **Harry, John et al,**
**BP TRADING LIMITED Patents & Licensing Division**
**Chertsey Road**
**Sunbury-on-Thames Middlesex TW16 7LN(GB)**

(54) Oil-in-water monitor.

(57) This invention relates to a measurement cell 1 suitable for monitoring the oil content of water-oil mixtures by measuring the attenuation of radiation incident upon a falling stream 10 of the mixture. The inventive feature lies in adapting the cell interior to enable provision of a gaseous curtain between the radiation source 14 and the falling stream 10 on the one hand and between the falling stream 10 and a detector 15 of attenuated radiation on the other hand. The provision of a gaseous curtain prevents window fouling at the radiation source and the detector thereby improving the accuracy of the measurement.

EP 0 026 093 A1

1

OIL-IN-WATER MONITOR

The present invention relates to a measurement cell suitable for monitoring the oil content of oil-in-water mixtures, especially mixtures encountered during the deballasting of oil tankers.

During deballasting, it is necessary to establish the amount of oil in the water being discharged to ensure that the mixture is channelled to the appropriate destination according to its oil content, hence minimising pollution hazards which may result by discharge of oil into the sea. Processes utilising the differences in optical properties of the liquids have been used to monitor the amount of oil present in an oil-in-water mixture, but have given variable and inaccurate results since the responses obtained by such devices are subject to the effects of scattered light resulting from the cell geometry and from cell window fouling.

It has now been found that these effects, particularly the effects of cell window fouling, may be reduced by a novel cell design.

Accordingly, the present invention is a device for monitoring the oil content of an oil-in-water mixture comprising a cell having:

(a) liquid inlet means adapted to produce a falling non-turbulent liquid stream across the cell,

(b) means for detecting the attenuation of incident radiation by the liquid comprising a source of electromagnetic radiation adapted to produce a beam of radiation passing through the liquid stream substantially at right angles, and a detector adapted to receive the attenuated radiation and to provide a signal proportional to the intensity of the radiation,

(c) gas flow means for providing a gaseous curtain between the radiation source and the liquid stream and between the detector and the liquid stream, the gaseous curtain being a current of gas flowing substantially parallel to but spaced from the liquid stream to prevent distortion of the latter, and

(d) gas/liquid outlet means.

The cell and its component parts are suitably made of metal or plastics material which do not allow ingress of unwanted scattered radiation and which are not adversely affected by the liquid flowing through the cell under ambient conditions.

The liquid inlet means are suitably shaped so as to produce a falling non-turbulent liquid stream across the cell. The form of the liquid stream is preferably cylindrical, but the liquid inlet means may be suitably shaped so as to produce a liquid stream in other forms for instance as a film provided that non-turbulent flow is maintained. The liquid inlet means may be in the form of detachable units so as to provide a variety of dimensions of the inlet for use over a wide range of oil concentrations.

It is important that the liquid mixture entering the cell during operation of the monitor is entirely homogeneous. The liquid mixture will normally be fed to the cell by means of a delivery pump and the pump is suitably connected to the liquid inlet means via a flow controller. The delivery pump, homogeniser and flow controller may be integral with the cell or detachable.

The liquid outlet means is suitably an elongated extension of the lower part of the cell which channels the falling liquid stream into a drain or other similar means of discharge.

The means for detecting the attenuation of incident radiation by the liquid comprises a radiation source and a detector which are suitably positioned so that the beam of radiation passes through the liquid stream at a point close to the liquid inlet means where the falling liquid stream is substantially free from turbulence. The electro-magnetic radiation may be visible white or mono-chromatic light of any wave length, ultra violet light or infra red radiation and may be polarised or unpolarised during its passage from the source to the detector.  Preferably the radiation source is one
which produces white or mono-chromatic light.  The detector may
be a conventional photo electric cell.  The light from the
radiation source is suitably projected and the attenuated light
received by the detector by means of fibre optic light guides.
These light guides may be housed in enclosures in the cell walls
which form windows in open communication with the cell interior.
The windows may however be covered with transparent material such
as glass or transparent polymer, and in this case the window
covering preferably has a flat or hemispherical surface adjacent to
the light guide and a substantially hemispherical surface adjacent
to the interior of the cell so that the window covering forms a
convex lens for the light emerging from and being received by the
fibre optic light guides.  The use of a gaseous curtain in the
device of the present invention, however, enables the cell to
function efficiently and continuously with or without such window
coverings.

The gas flow means for providing a gaseous curtain suitably
comprises an annular opening in the top of the cell surrounding the
liquid inlet means and adapated to provide a cylindrical gaseous
curtain concentric with and flowing in a direction substantially
parallel to the falling liquid stream.  Alternatively the gas flow
means may comprise two openings in the top of the cell on either side
of the liquid inlet means so as to provide two gaseous curtains on
either side of the falling liquid stream and flowing in a direction
substantially parallel to this to protect the radiation source on the
one hand and the detector on the other hand.  The flow of gas forming
the gaseous curtain is spaced from the falling liquid stream, in order
to prevent any distortion of this.  The gas which in operation is
pumped into the cell to provide the gaseous curtain is suitably inert
not only with respect to the oil in water mixture but should also be
non-corrosive to the cell structure.  Examples of suitable gases are
air, nitrogen and argon.

In a preferred embodiment of the device according to the present
invention the cell interior below the cell windows is recessed to
minimise any turbulence created by the gaseous curtain.

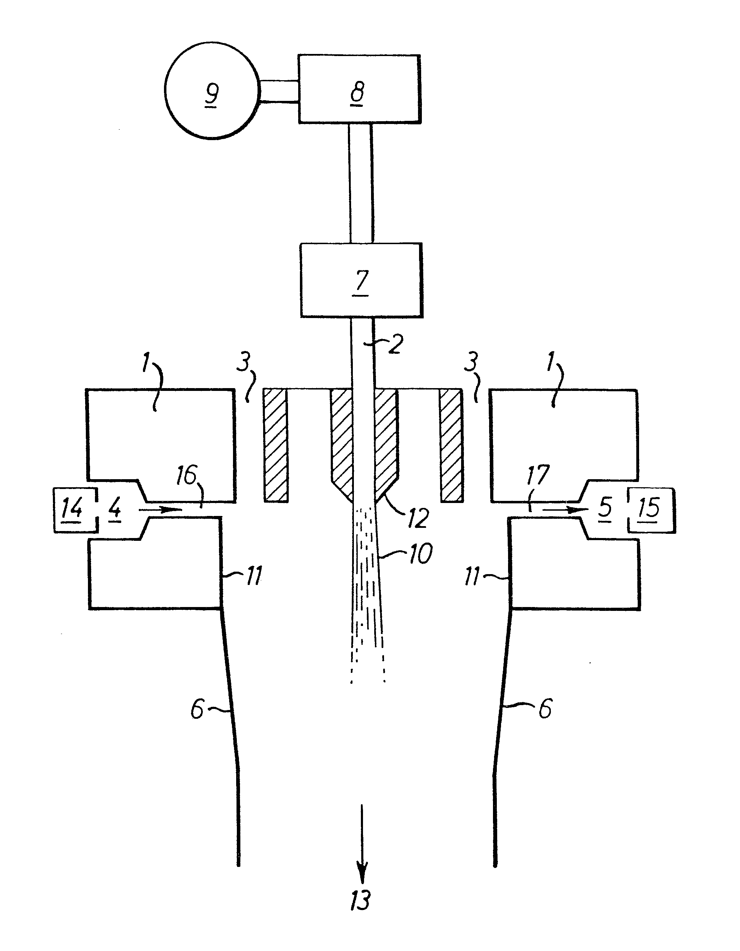

The invention is further illustrated with reference to the accompanying drawing which is a view of the cell in vertical cross-section.

In the drawing, the cell housing 1 is provided with a liquid inlet pipe 2 which has a tapered exit 12 allowing a non-turbulent flow of liquid to emerge therefrom. The inlet pipe is connected to a pump 9 through a homogeniser 8 and a flow controller 7. Surrounding the inlet pipe 2 is an annular gas inlet 3 which allows a current of gas to be admitted into the cell in the form of cylindrical curtain of gas surrounding the falling liquid stream. The cell housing 1 is provided on either side with apertures 4 and 5 for introducing electromagnetic radiation into and detecting attenuated radiation emerging from the cell by means of radiation source 14 and detector 15 respectively. The apertures 4 and 5 are positioned in a line substantially at right angles to and as close to the tapered exit 12 as possible. The radiation source 14 and the detector 15 are provided with light guides 16 and 17 to direct the radiation into and from the cell. The lower half of the cell housing below the apertures is recessed at 11 to prevent distortion of the non-turbulent liquid flow. In order to prevent ingress of any stray radiation, the lower half of the cell is provided with shaped guards 6 which also assist in maintaining the gaseous currents substantially parallel to the falling liquid stream 10. The falling liquid stream 10 and the gaseous currents are led out of the cell through the outlet means 13.

In operation, a current of air is admitted through the gas inlets 3 into the cell. The rate of flow of air into the cell is adjusted to that compatible with the desired flow rate of the oil-in-water mixture into the cell. Before admission of any liquid into the cell but with the current of air flowing through the cell a beam of light from the radiation source 14 is projected into the cell through aperture 4 by means of light guide 16. The attenuation, if any, by the current of air in the cell is detected by the detector 15 through aperture 5 by means of light guide 17 connected to detector 15. A similar reading is taken using a falling stream of pure water. These readings are

**0026093**

used to calibrate the apparatus. The oil-in-water mixture under examination is then pumped by pump 9 via the homogeniser 8 and flow controller 7 into the inlet pipe 2. The variation in intensity of the light passing through the liquid mixture emerging from the exit 12 is detected as before using the detector 15. The output voltage of the detector 15 is fed into a logarithmic amplifier which will provide a linear relation between the output and contaminant oil concentration in the liquid mixture assuming that the particle size distribution of the contaminant remains constant over the range of concentrations.

The principal advantages of this invention are that it allows continuous and efficient operation of the cell without fear of splashing of the radiation source or the detector enclosures and substantially reduces 'ageing' due to oil mist coating of these enclosures. This is particularly important in cases where the oil concentration of the mixture is above 500 ppm. The present cell enables detection of oil concentrations up to 5000 ppm accurately because the gaseous curtain keeps the radiation source and detector enclosures substantially clean.

Claims:

1. A device for monitoring the oil content of an oil-in-water mixture comprising a cell having:

(a) liquid inlet means adapted to produce a falling non-turbulent liquid stream across the cell,

(b) means for detecting the attenuation of incident radiation by the liquid comprising a source of electromagnetic radiation adapted to produce a beam of radiation passing through the liquid stream substantially at right angles, and a detector adapted to receive the attenuated radiation and to provide a signal proportional to the intensity of the radiation,

(c) gas flow for providing a gaseous curtain between the radiation source and the liquid stream and between the detector and the liquid stream, the gaseous curtain being a current of gas flowing substantially parallel to but spaced from the liquid stream to prevent distortion of the latter, and

(d) gas/liquid outlet means.

2. A device according to claim 1 wherein the liquid inlet means are shaped so as to produce a falling non-turbulent liquid stream across the cell.

3. A device according to claim 2 wherein the non-turbulent liquid stream is cylindrical.

4. A device according to any one of the preceding claims wherein the liquid inlet means is in the form of detachable units.

5. A device according to any one of the preceding claims wherein the liquid outlet means is an elongated extension of the lower part of the cell.

6. A device according to any one of the preceding claims wherein the radiation source and detector are positioned so that the beam of radiation from the source passes the liquid stream at a point close to the liquid inlet means.

7. A device according to any one of the preceding claims wherein the source of electromagnetic radiation is visible white or mono-chromatic light.

8. A device according to claim 7 wherein the detector is a photo-electric cell.

9. A device according to any one of the preceding claims wherein the detector receives attenuated radiation through fibre-optic light guides.

10. A device according to any one of the preceding claims wherein the radiation source and the detector are housed in enclosures in the cell walls, said enclosures being in the form of windows which are either in open communication with or are separated by a transparent cover from the cell interior.

European Patent Office

**EUROPEAN SEARCH REPORT**

Application number

EP 80 30 3306

| | DOCUMENTS CONSIDERED TO BE RELEVANT | | CLASSIFICATION OF THE APPLICATION (Int Cl.³) |
|---|---|---|---|
| Category | Citation of document with indication, where appropriate, of relevant passages | Relevant to claim | G 01 N 21/85 |
| | GB - A - 1 467 312 (K. HAYASHI) <br> * Page 3; figure 1 * <br> -- | 1,2,6, 8 | |
| | PATENTS ABSTRACTS OF JAPAN, vol. 1, no. 88, 16th August 1977, page 2023E77 <br> & JP - A - 52 22978 (TOEI DENSHI KOGYO) <br> * Whole document * <br> -- | 1,8 | |
| | FR - A - 2 123 948 (F. GUIGUES) <br> * Pages 2,3; figures 4,5 * <br> -- | 1,8-10 | TECHNICAL FIELDS SEARCHED (Int. Cl.³) <br><br> G 01 N 21/85 <br> 21/15 <br> 33/18 <br> 21/53 <br> 15/06 |
| | US - A - 3 628 028 (J. THORSHEIM) <br> * Columns 1,2; figure 1 * <br> -- | 1,10 | |
| | US - A - 3 702 403 (H. KISHI) <br> * Columns 3,4; figure 1 * <br> -- | 1 | |
| A | US - A - 3 899 688 (J. PERIERES) <br> * Column 4; figure 3 * <br> -- | 1,8-10 | |
| A | DE - B - 1 598 353 (HOWALDTSWERKE DEUTSCHE WERFT) <br> * Column 2; figures 1,2 * <br> ---- | 1,10 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 30-12-1980 | BOEHM |

EPO Form 1503.1 06.78